# EUROPEAN PATENT APPLICATION

(11) **EP 4 578 389 A2**
(43) Date of publication of application: **02.07.2025**
(21) Application number: 25176994.9
(22) Date of filing: 30.12.2022
(51) Int. Cl.: A61B 5/287

(54) **RECONFIGURABLE ELECTRODE APPARATUS FOR DIAGNOSIS OF ARRHYTHMIAS**

(30) Priority: 31.12.2021 US 202163295702 P; 17.11.2022 US 202218056670
(62) Divisional of application: 22217252.0
(71) Applicant: Biosense Webster (Israel) Ltd., 2066717 Yokneam (IL)
(72) Inventor: ABBAS, Mohammad, Irvine, 92618 (US); TOBEY, Dustin R., Irvine, 92618 (US); EBRAHIMI, Babak, Irvine, 92618 (US)
(74) Representative: Carpmaels & Ransford LLP

(57) **Abstract**

Example apparatuses disclosed herein are generally usable with catheter-based systems to measure or provide electrical signals within the heart and surrounding vasculature. Example apparatuses generally include an end effector with one or more spines that can rotate about a longitudinal axis such that the spines are aligned in a plane in a first configuration and the one or more spines are rotated out of the plane in a second configuration. The end effector can include features which provide improved and/or alternative diagnostic or treatment options compared to existing end effectors. In some example treatments utilizing some example apparatuses presented herein, an end effector can map a wall within the heart in the first configuration and a lumen of a vein in the second configuration.

## Description

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to provide high-density signal maps through the use of several electrodes sensing electrical activity of tissue in an area on the order of a square centimeter. For mapping within an atria or a ventricle (for example, an apex of a ventricle), it is desirable for a catheter to collect larger amounts of data signals within shorter time spans. It is also desirable for such a catheter to be adaptable to different tissue surfaces, for example, flat, curved, irregular or nonplanar surface tissue and be collapsible for atraumatic advancement and withdrawal through a patient's vasculature.

### SUMMARY

Example apparatuses disclosed herein are generally usable with catheter-based systems to measure or provide electrical signals within the heart and surrounding vasculature. Example apparatuses generally include an end effector with one or more spines that can rotate about a longitudinal axis such that the spines are aligned in a plane in a first configuration and the one or more spines are rotated out of the plane in a second configuration. The end effector can include features which provide improved and/or alternative diagnostic or treatment options compared to existing end effectors. In some example treatments utilizing some example apparatuses presented herein, an end effector can map a wall within the heart in the first configuration and a lumen of a vein in the second configuration.

An example end effector for use with a catheter can include a tubular shaft and a plurality of spines. The tubular shaft can extend along a longitudinal axis. The plurality of spines can be disposed on a plane contiguous to the plurality of spine and the longitudinal axis in a first configuration. At least one spine can be configured for rotation about the longitudinal axis such that the at least one spine is disposed outside of the plane in a second configuration.

Each spine can extend generally parallel to the longitudinal axis. Alternatively, each spine can extend at angle with reference to the longitudinal axis.

Another example apparatus can include an elongated shaft, a handle, and an end effector. The elongated shaft can include a proximal portion and a distal portion. The elongated shaft can be configured to be manipulated at the proximal portion to position the distal portion into a heart of a patient. The elongated shaft can define a longitudinal axis of the apparatus. The handle can be affixed to the proximal portion of the shaft. The end effector can be disposed proximate the distal portion of the elongated shaft. The end effector can include a plurality of spine pairs. Each spine includes electrodes disposed thereon. The plurality of spine pairs can include an outer pair of spines and an inner pair of spines such that in a first configuration, the outer pair of spines are contiguous to a plane with the inner pair of spines, such that in a second configuration, one of the inner pair of spines or the outer pair of spines are rotated about the longitudinal axis so that the one pair of spines is out of alignment with the other pair of spines.

Each pair of spines includes a connecting member to define a spine loop so that the plurality of spine pairs define a plurality of spine loops. At least one of the plurality of spine loops can be rotatable about the longitudinal axis.

Each of the spine loops can include a substantially rectangular shape. Alternatively, each of the spine loops can include a substantially trapezoidal shape having a distal width wider than a proximal width.

The plurality of spine loops can include three spine loops. At least two of the three spine loops can be rotatable about the longitudinal axis by manipulation of the handle.

The end effector can include a first spine loop including the outer pair of spines. The first spine loop can define an outer perimeter of the end effector when the plurality of spines are in the first configuration. A second spine loop can include the inner pair of spines. The inner pair of spines can be positioned between the outer pair of spines when the plurality of spines are in the first configuration. A third spine loop can include a central pair of spines of the plurality of spines. The central pair of spines can be positioned between the inner pair of spines when the plurality of spines are in the first configuration.

The first spine loop can be configured to rotate between 30° and 90° about the longitudinal axis. The second spine loop can be configured to rotate between -30° and -45° about the longitudinal axis. In the second configuration, the first spine loop can be at an angle of approximately 60° to the second spine loop, the second spine loop can be at an angle of approximately 60° to the third spine loop, and the third spine loop can be at an angle of approximately 60° to the first spine loop.

The end effector can further include a flexible linkage affixed to the plurality of spine loops at a distal end of the end effector along the longitudinal axis.

The end effector can further include a support linkage affixed to a distal end of at least one of the spine loops of the plurality of spine loops, extending along the longitudinal axis, and affixed to the distal portion of the elongated shaft.

The spines can be positioned in the second configuration when the end effector is in free space. The spines can be movable to the first configuration when the end effector is pressed against a planar surface.

The apparatus can further include a pull wire extending from the handle to the distal portion of the elongated shaft such that manipulation of the handle moves the pull wire and causes the plurality of spines to move between the second configuration and the first configuration. A pull wire can be made from any suitable materials that can endure tight bends while still allowing for movement. For example, high molecular weight polymer or ultra-high molecular weight polymer (e.g., Vectran, Spectra) can be used as well as nylon for a wire.

The apparatus can further include a first rotating frame disposed in the distal portion of the elongated shaft and affixed to a first spine of the plurality of spines. The first rotating frame and the first spine can be configured to rotate about the longitudinal axis in response to movement of the pull wire.

The first rotating frame can be affixed to proximal ends of a first spine loop. The first spine loop can include the first spine and a second spine of the outer pair of spines. The first rotating frame and the first spine loop can be configured to rotate about the longitudinal axis in response to movement of the pull wire.

The apparatus can further include a second rotating frame disposed in the distal portion of the elongated shaft and affixed to proximal ends of a second spine loop. The second spine loop can include the inner pair of spines. The second rotating frame and the second spine loop can be configured to rotate about the longitudinal axis in response to movement of the pull wire. The first rotating frame and the first spine loop can be configured to rotate oppositely about the longitudinal axis in relation to rotation of the second rotating frame and the second spine loop about the longitudinal axis. The first spine loop can include a first support frame affixed to the first rotating frame. The second spine loop can include a second support frame affixed second rotating frame.

The apparatus can further include a distal cap affixed at the distal portion of the elongated shaft, distal of the first rotating frame and the second rotating frame. A distal end of the pull wire can be affixed to the distal cap. The pull wire can be threaded through a first pull wire lumen of the first rotating frame. The pull wire can be threaded through a second pull wire lumen of the second rotating frame. Tension in the pull wire can move the first pull wire lumen and the second pull wire lumen into alignment.

The apparatus can further include a plurality of pull wires each affixed to the distal cap and each threaded through a first respective pull wire lumen of the first rotating frame and a second respective pull wire lumen of the second rotating frame. The plurality of pull wires can be configured to move the plurality of spines from the planar shape to the second configuration and from the second configuration to the first configuration.

The apparatus can further include a center column disposed in the distal portion of the elongated shaft and affixed to proximal ends of a third spine loop. The third spine loop can include a central pair of spines. The first rotating frame and/or the second rotating frame can be movable to rotate about the center column in response to movement of the pull wire.

The apparatus can further include a plurality of electrical conductors extending through the elongated shaft, through the center column, and electrically connected to the electrodes.

The distal portion of the elongated shaft can be configured to deflect in relation to longitudinal axis in response to manipulation of the handle.

An example method can include one or more of the following steps presented in no particular order. A distal portion of an elongated shaft and an end effector extending distally from the distal portion of the elongated shaft can be moved, through a catheter to a heart. The end effector can include a plurality of spines with electrodes thereon. The plurality of spines can include an outer pair of spines and an inner pair of spines. The elongated shaft can define a longitudinal axis. The end effector can be moved from a distal end of the catheter via manipulation of a proximal portion of the elongated shaft. The end effector can be positioned against a wall within the heart such that a majority of a length of each spine of the plurality of spines conforms to the wall. At least a portion of the end effector can be positioned within a vein. A portion of the length of each spine of the plurality of spines can be apposed to the vein.

The method can further include rotating, about the longitudinal axis, the inner pair of spines and/or the outer pair of spines such that the inner pair of spines is non-coplanar to the outer pair of spines.

The method can further include manipulating a handle at a proximal end of the elongated shaft to thereby rotate a spine loop including the inner pair of spines or the outer pair of spines about the longitudinal axis.

The method can further include rotating the spine loop between 30° and 90° about the longitudinal axis.

The method can further include twisting a flexible linkage affixed to a plurality of spine loops at a distal end of the end effector along the longitudinal axis as the spine loop is rotated.

The method can further include positioning the spines in a basket shape when the end effector is in free space within the heart. The method can further include pressing the end effector against the wall to move the spines to a planar shape.

The method can further include manipulating a handle at the proximal portion of the elongated shaft to move a pull wire extending from the handle to the distal portion of the elongated shaft to thereby cause the plurality of spines to move between a basket shape and a planar shape.

The method can further include rotating, about the longitudinal axis, in response to movement of the pull wire, a first rotating frame disposed in the distal portion of the elongated shaft and affixed to a first spine of the plurality of spines.

The method can further include rotating, about the longitudinal axis, a first spine loop including the first spine and a second spine, the first rotating frame being affixed to proximal ends of a first spine loop.

The method can further include rotating, about the longitudinal axis, in response to movement of the pull wire, a second rotating frame and a second spine loop, the second rotating frame being disposed in the distal portion of the elongated shaft and affixed to proximal ends of the second spine loop, the second spine loop including a third spine and a fourth spine.

The method can further include rotating the first rotating frame and the first spine loop oppositely about the longitudinal axis in relation to rotation of the second rotating frame and the second spine loop about the longitudinal axis.

The method can further include providing tension to the pull wire to move a first pull wire lumen of the first rotating frame into alignment with a second pull wire lumen of the second rotating frame, the pull wire being threaded through the first pull wire lumen and the second pull wire lumen.

The method can further include manipulating a plurality of pull wires to move the plurality of spines from the planar shape to the basket shape and from the basket shape to the planar shape, each of the plurality of pull wires being affixed to a distal cap and each threaded through a first respective pull wire lumen of the first rotating frame and a second respective pull wire lumen of the second rotating frame.

The method can further include rotating the first rotating frame and/or the second rotating frame about a center column disposed in the distal portion of the elongated shaft and affixed to proximal ends of a third spine loop, the third spine loop comprising a fifth spine and a sixth spine.

The method can further include mapping electrical signals of the wall with the electrodes, and mapping electrical signals of the vein with the electrodes.

The method can further include deflecting the distal portion of the elongated shaft in relation to longitudinal axis in response to manipulation of the handle.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
Figure 1 is an illustration of a catheter having an end effector in a first configuration at a distal portion of the catheter and a proximal handle at a proximal portion of the catheter according to aspects of the present invention.
Figure 2A is a perspective illustration of the end effector in a second configuration according to aspects of the present invention.
Figure 2B is a distal end illustration of the end effector of Figure 2A.
Figure 3 is an illustration of the end effector positioned to map a wall within a heart according to aspects of the present invention.
Figure 4 is an illustration of the end effector position to map a lumen of a vein according to aspects of the present invention.
Figures 5A, 5B, 6A, and 6B are illustrations of various views of an alternative end effector having trapezoidal spine loops in a first configuration according to aspects of the present invention.
Figures 7A and 7B are illustrations of various views of the alternative end effector having trapezoidal spine loops in a second configuration according to aspects of the present invention.
Figure 8 is an illustration of support frames of the alternative end effector having trapezoidal spine loops in the first configuration according to aspects of the present invention.
Figures 9A and 9B are illustrations of another alternative end effector having spines with free ends in a first configuration according to aspects of the present invention.
Figures 10A and 10B are illustrations of the alternative end effector having spines with free ends in a second configuration according to aspects of the present invention.
Figure 11A is an illustration of a distal end view of a distal portion of the elongated shaft of an example apparatus in a first configuration according to aspects of the present invention.
Figure 11B is an illustration of a distal end view of the distal portion of the elongated shaft of the example apparatus illustrated in Figure 11A in a second configuration according to aspects of the present invention.
Figure 12A is an illustration of rotating frames of the distal portion of the elongated shaft in a first configuration according to aspects of the present invention.
Figure 12B is an illustration of rotating frames of the distal portion of the elongated shaft in a second configuration according to aspects of the present invention.
Figure 13A is an illustration of the distal portion of the elongated shaft according to aspects of the present invention.
Figure 13B is an illustration of the distal portion of the elongated shaft in Figure 13A with a distal cap removed according to aspects of the present invention.
Figure 14 is an illustration of an example flow diagram of a method for mapping electrical signals through cardiac anatomy according to aspects of the present invention.

### DETAILED DESCRIPTION

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the pertinent art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the pertinent art in view of the teachings herein. Such modifications and variations are intended to be included within the scope of the claims.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±25% of the recited value, e.g. "about 90%" may refer to the range of values from 65% to 115%.

As used herein, the terms "patient," "host," "user," and "subject" refer to any human or animal subject and are not intended to limit the systems or methods to human use, although use of the subject invention in a human patient represents a preferred embodiment.

Examples illustrated herein generally relate to catheters with end effectors which can be shaped in a first configuration to map electrical signals of a substantially planar surface such as a wall within a heart and shaped in a second configuration to map electrical signals around a bodily lumen such as within a vein.

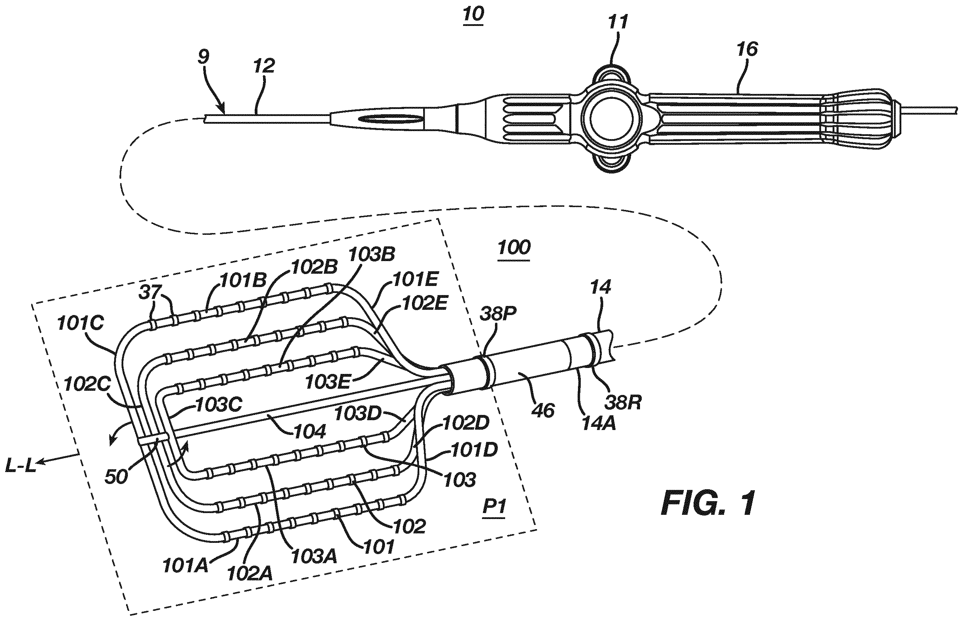

Figure 1 illustrates an example apparatus 10 having an elongated shaft 9, a distal electrode assembly or end effector 100, and a deflection control handle 16. The apparatus 10 can have several design variations while including novel aspects illustrated herein. The apparatus 10 is presented for illustration purposes only and is not intended to be limiting.

The elongated shaft 9 has a proximal portion 12 in the shape of an elongated catheter body, an intermediate deflection section 14, and distal portion 14A. The deflection control handle 16 is attached to the proximal end of the catheter body 12. Suitable control handles are disclosed in U.S. Patent Nos. 6,123,699; 6,171,277; 6,183,435; 6,183,463; 6,198,974; 6,210,407 and 6,267,746, the entire disclosures of which are incorporated herein by reference and attached in the Appendix of priority application U.S. 63/295,702. The distal portion 14A of the shaft is coupled to the end effector 100 via a connector tubing 46. The elongated shaft 9 forms a tubular catheter body sized and otherwise configured to traverse vasculature. The end effector 100 has a plurality of spine loops 101, 102, 103. The spine loops can be joined at a distal vertex of the end effector 100 with a mechanical linkage 50. The end effector 100 can include a support linkage 104 extending along the longitudinal axis L-L, affixed to a distal end of at least one of the spine loops and affixed to the distal portion 14A of the elongated shaft 9.

When the device is unconstrained and aligned, the proximal portion 12, intermediate section 14, distal portion 14A, and end effector 100 are generally aligned along the longitudinal axis L-L. The intermediate section 14 can be configured to bend to deflect the distal portion 14A and end effector 100 from the longitudinal axis L-L.

The end effector 100 can be collapsed (compressed toward the longitudinal axis L-L) to fit within a guiding sheath or catheter (not illustrated). The shaft 9 can be pushed distally to move the end effector 100 distally through the guiding sheath. The end effector 100 can be moved to exit a distal end of the guiding sheath via manipulation of the shaft 9 and/or control handle 16. An example of a suitable guiding sheath for this purpose is the Preface Braided Guiding Sheath, commercially available from Biosense Webster, Inc. (Irvine, California, USA).

The end effector 100 has first, second and third spine loops 101, 102, and 103. Each spine loop 101, 102, 103 has two spines 101A, 101B, 102A, 102B, 103A, 103B and a connector 101C, 102C, 103C that connects the two spines of the respective spine loop 101, 102, 103. Spines 101A, 101B of a first spine loop 101 are connected by a first connector 101C; spines 102A, 102B of a second spine loop 102 are connected by a second connector 102C; and spines 103A, 103B of a third spine loop 103 are connected by a third connector 103C. Each spine loop 101, 102, 103 further includes proximal segments 101D, 101E, 102D, 102E, 103D, 103E joining to the distal portion 14A of the shaft 9.

For each spine loop 101, 102, 103, the spines 101A, 101B, 102A, 102B, 103A, 103B in the respective pair of spines can be substantially parallel to each other along a majority of their respective lengths when the end effector 100 is expanded in an unconstrained configuration as illustrated in Figure 1. Preferably, all spines in the end effector are parallel to each other along the majority of their respective lengths when the end effector 100 is in the unconstrained configuration. Preferably, all spines are coplanar or approximately coplanar, defining a plane P1 when the end effector is in a first configuration as illustrated in Figure 1. One or more of the spines 101A, 101B, 102A, 102B, 103A, 103B can be rotated about the longitudinal axis L-L and out of the plane P1 when the end effector is in a second configuration.

The end effector 100 can optionally include a flexible linkage 50 joining the spine loops 101, 102, 103 at a distal vertex of the end effector aligned with the longitudinal axis L-L. The flexible linkage 50 can function to maintain spatial relation of the spine loops 101, 102, 103 at the distal vertex and be flexible to allow rotation of one or more of the spine loops 101, 102, 103.

The end effector 100 can optionally include a support linkage 104 affixed to a distal end of at least one of the spine loops 101, 102, 103, extend along the longitudinal axis L-L, and affixed at a distal portion of the elongated shaft. The support linkage 104 can provide additional structural support to the end effector 100.

Each spine 101A, 101B, 102A, 102B, 103A or 103B can have a length ranging between about 5 and 50 mm, preferably about 10 and 35 mm, and more preferably about 28 mm. The parallel portions of each spine 101A, 101B, 102A, 102B, 103A, 103B can be spaced apart from each other by a distance ranging between about 1mm and 20 mm, preferably about 2 and 10 mm, and more preferably about 4 mm. Each spine 101A, 101A, 101B, 102A, 102B, 103A, 103B preferably carries at least eight electrodes per spine member. The end effector preferably includes six spines as illustrated. With eight electrodes on six spines, the end effector 100 includes forty-eight electrodes.

A distal electrode 38D and a proximal electrode 38P are positioned near the distal portion 14A of the shaft 9. The electrodes 38D and 38P can be configured to cooperate (e.g. by masking of a portion of one electrode and masking a different portion on the other electrode) to define a referential electrode (an electrode that is not in contact with tissues). One or more impedance sensing electrodes 38R can be configured to allow for location sensing via impedance location sensing technique, as described in U.S. Patent Nos. 5,944,022; 5,983,126; and 6,445,864, which are incorporated by reference herein and attached to the Appendix of priority application U.S. 63/295,702.

The catheter body 12 can be flexible, i.e., bendable, but substantially non-compressible along its length. The catheter body 12 can be of any suitable construction and made of any suitable material. In some embodiments, the catheter body 12 has an outer wall made of polyurethane or PEBAX. The outer wall may include an imbedded braided mesh of stainless steel or the like to increase torsional stiffness of the catheter body 12 so that, when the control handle 16 is rotated, the intermediate section 14 will rotate in a corresponding manner. The outer diameter of the catheter body 12 is preferably no more than about 8 French, more preferably about 7 French.

The catheter body 12 can include, extending therethrough: a plurality of lead wires for ring electrodes 37 carried on the spines 101A, 101B, 102A, 102B, 103A, 103B; one or more puller wires to bend the intermediate portion 14; a cable for an electromagnetic position sensor positioned within the distal portion 14A; and lead wires for distal and proximal ring electrodes 38D, 38P carried on the catheter proximally of the end effector 100. Electromagnetic location sensing technique is described in U.S. Patent Nos. 5,391,199; 5,443,489; 5,558,091; 6,172,499; 6,590,963; and 6,788,967 which are incorporated by reference herein and attached to the Appendix of priority application U.S. 63/295,702. The magnetic location sensor can be utilized with impedance sensing electrode 38R in a hybrid magnetic and impedance position sensing technique known as ACL described in U.S. Patent Nos. 7,536,218; 7,756,567; 7,848,787; 7,869,865; and 8,456,182, which are incorporated by reference herein and attached to the Appendix of priority application U.S. 63/295,702.

The useful length of the catheter shaft 9, i.e., that portion of the apparatus 10 that can be inserted into the body excluding the end effector, can vary as desired. Preferably the useful length ranges from about 110 cm to about 120 cm. The length of the intermediate section 14 is a relatively smaller portion of the useful length, and preferably ranges from about 3.5 cm to about 10 cm, more preferably from about 5 cm to about 6.5 cm.

The distal portion 14A of the shaft 9 can be substantially contiguous with the intermediate section 14 such that the intermediate section comprises the distal portion 14A; the distal portion being distinguished from the intermediate section 14 by the positioning of one or more (optional) ring electrodes 38R. As referred to herein, the distal portion 14A of the shaft 9 can therefore correspond to a distal portion of the intermediate section 14.

The distal portion 14A of the shaft 9 can be coupled to the end effector 100 with a connector tubing 46. The connector tubing 46 includes an insert for connection of spine loops 101, 102, 103 to provide electrical connection through the intermediate portion 14 of the catheter body. The connector tubing 46 can be affixed to the distal portion 14A of the catheter by glue or the like.

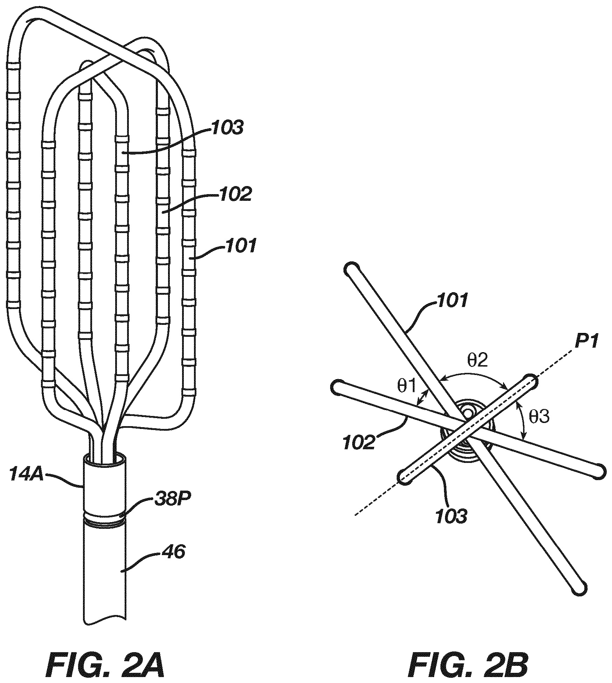

Figures 2A and 2B are illustrations of the end effector 100 in a second configuration. The optional flexible linkage 50 and support linkage 104 are omitted in Figures 2A and 2B. An outer spine loop 101 and an inner spine loop 102 are rotated out of the plane P1 in the second configuration. A central spine loop 103 is maintained in the plane P1. Alternatively, only the outer spine loop 101 or the inner spine loop 102 can be rotated out of the plane P1. As another alternative, all three spine loops 101, 102, 103 can be rotated out of the plane P1.

As shown in Figure 2B, the inner spine loop 102 is at an angle θ1 to the outer spine loop 101; the outer spine loop 101 is at an angle θ2 to the central spine loop 103; and the central spine loop 103 is at an angle θ3 to the inner spine loop 102. The angles θ1, θ2, θ3 preferably each measure between 30° and 90°. More preferably, the angles θ1, θ2, θ3 each measure approximately 60°. As illustrated in Figure 2B, the angle θ1 between the inner spine loop 102 and the outer spine loop 101 measures approximately 45°; the angle θ2 between the outer spine loop 101 and the central spine loop 103 measures about 90°; and the angle θ3 between the central spine loop 103 and the inner spine loop 102 measures about 45°. Note that in some configurations, the sum of angles θ1, θ2, θ3 can equal 180°.

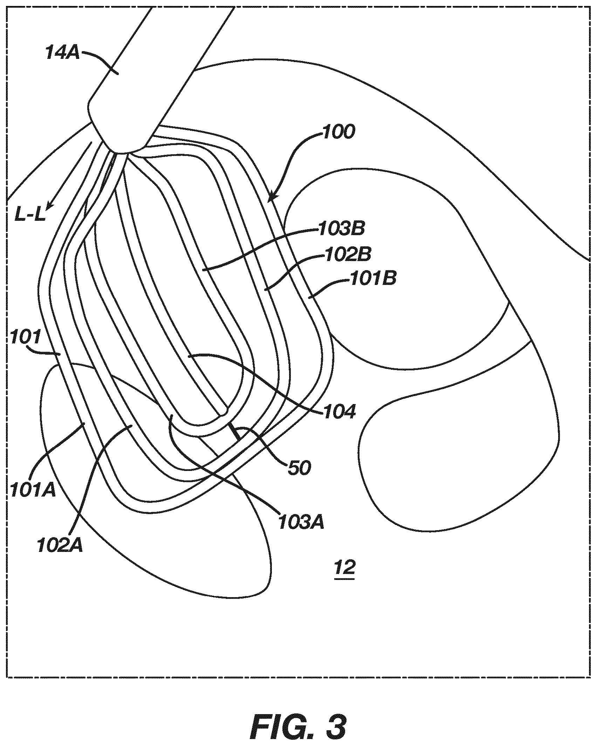

Figure 3 is an illustration of the end effector 100 positioned to map a wall within a model of a heart 20 in the first configuration. The spines 101A, 101B, 102A, 102B, 103A, 103B are aligned approximately coplanar to conform to the wall of the heart 20.

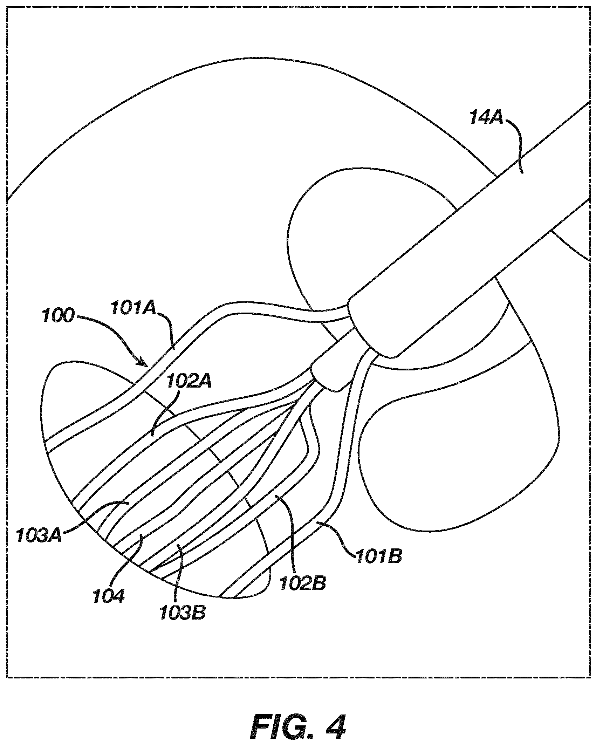

Figure 4 is an illustration of the end effector 100 position to map a lumen of a pulmonary vein in the second configuration. Preferably at least a portion of each spine 101A, 101B, 102A, 102B, 103A, 103B is in contact with the lumen of the vein, so that the lumen is contacted circumferentially at six positions. Alternatively, at least a portion of outer spines 101A, 101B of the outer spine loop 101 and at least a portion of the inner spines 102A, 102B of the inner spine loop 102 contact with the lumen of the vein, so that the lumen is contact circumferentially at four positions.

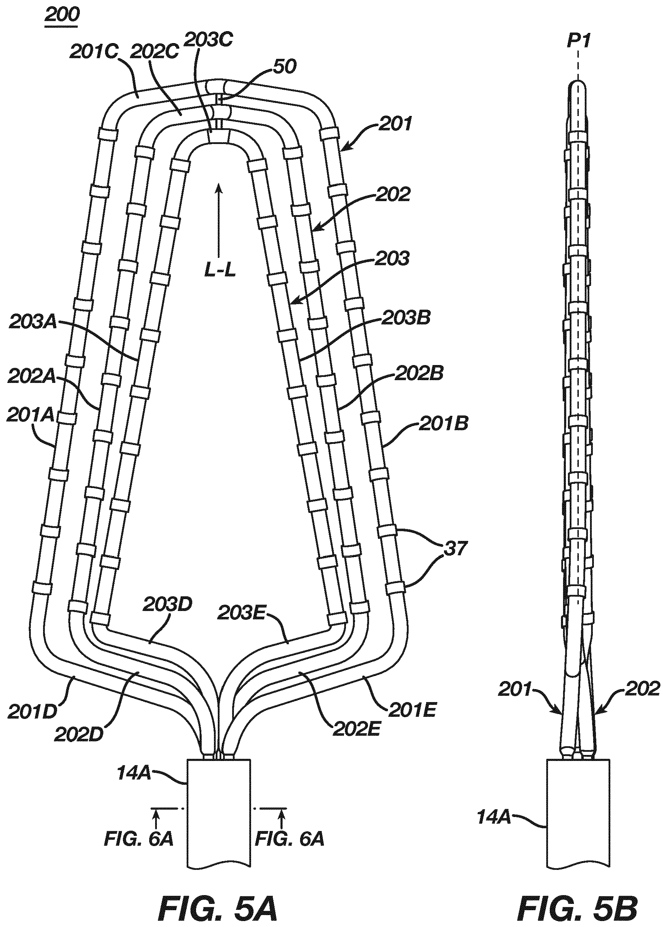

Figure 5A is a front view of an alternative end effector 200 having trapezoidal spine loops 201, 202, 203 in the first configuration. The end effector 200 illustrated in Figure 5A can be used in place of the end effector 100 illustrated in Figure 1 to modify the catheter 10 illustrated in Figure 1. The end effector 200 includes an outer spine loop 201, an inner spine loop 202, and a central spine loop 203. Each spine loop 201, 202, 203 includes a respective pair of spines 201A, 201B, 202A, 202B, 203A, 203B. Each spine loop 201, 202, 203 includes a connecting member 201C, 202C, 203C joining distal end of the respective pair of spines 201A, 201B, 202A, 202B, 203A, 203B. Each spine loop 201, 202, 203 includes a pair of proximal segments 201D, 201E, 202D, 202E, 203D, 203E joining the spine loop 201, 202, 203 to the distal portion 14A of the shaft 9. The spines 201A, 201B, 202A, 202B, 203A, 203B are further from the longitudinal axis L-L near a proximal end of the end effector 200 and closer to the longitudinal axis L-L near a distal end of the end effector 200 so that the spine loops 201, 202, 203 form a trapezoidal shape.

Figure 5B is a side view of the end effector 200 illustrated in Figure 5A in the first configuration. The spines 201A, 201B, 202A, 202B, 203A, 203B are approximately coplanar to define a plane P1.

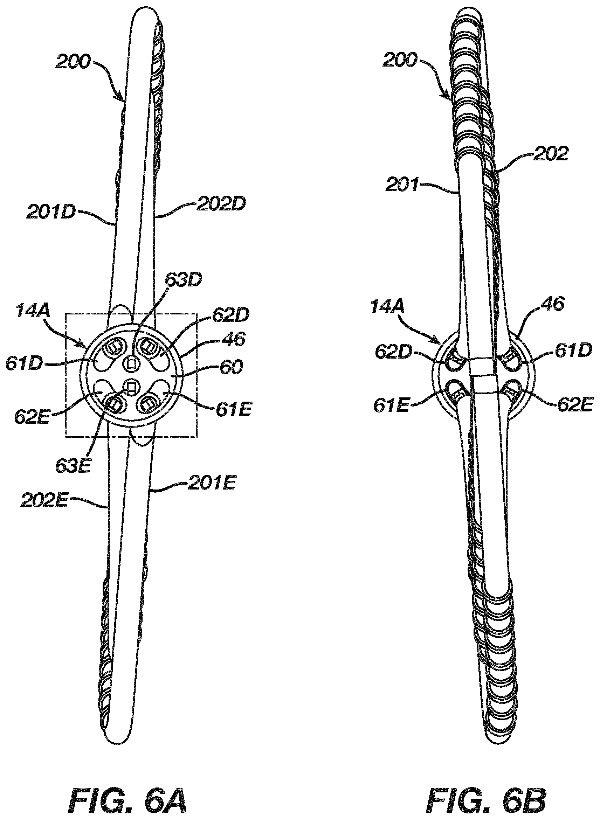

Figure 6A is a cross-sectional view of the distal portion 14A of the shaft 9 as indicated in Figure 5A. The distal portion 14A of the shaft 9 includes a distal cap 60. The distal cap includes: a first pair of openings 61D, 61E to receive proximal portions 201D, 201E of the outer spine loop 201; a second pair of openings 62D, 62E to receive proximal portions 202D, 202E of the inner spine loop 202; and a third pair of openings 63D, 63E to receive proximal portions 203D, 203E of the central spine loop 203. The first pair of openings 61D, 61E have an arcuate shape to allow the outer spine loop 201 to rotate about the longitudinal axis L-L. The second pair of openings 62D, 62E have an arcuate shape to allow the inner spine loop 201 to rotate about the longitudinal axis L-L. The third pair of openings 63D, 63E are shaped to inhibit rotation of the central spine loop 203 about the longitudinal axis L-L.

Figure 6B is an illustrations of a distal end view of the end effector 200 illustrated in Figures 5A, 5B, and 6A.

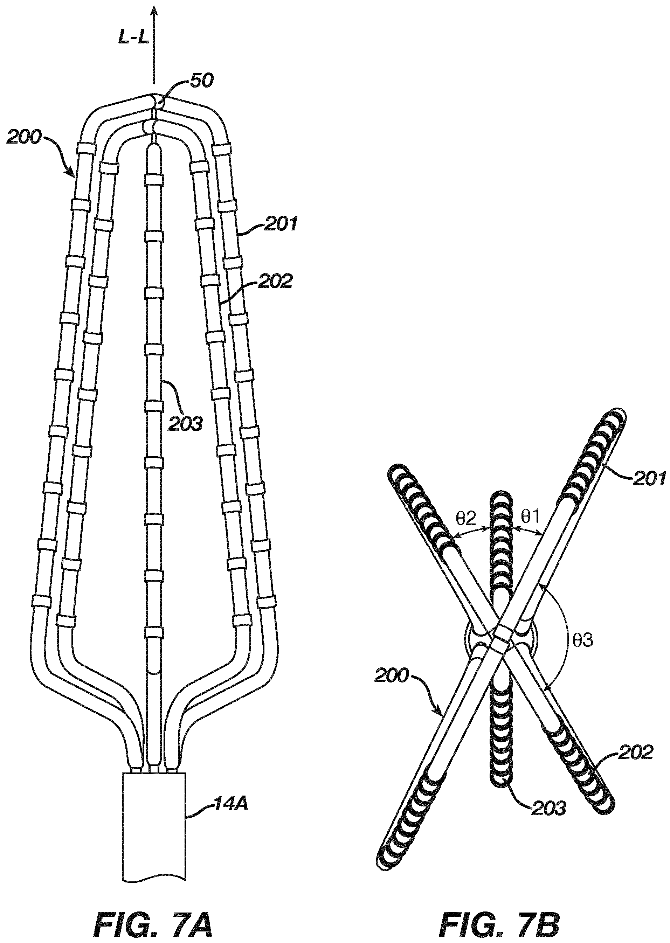

Figure 7A is an illustration of the end effector 200 in a second configuration in which the outer spine loop 201 and the inner spine loop 202 are each rotated about the longitudinal axis L-L.

Figure 7B is an illustration of a distal end view of the end effector in the second configuration. The inner spine loop 202 is at an angle θ1 to the outer spine loop 201; the outer spine loop 201 is at an angle θ2 to the central spine loop 203; and the central spine loop 203 is at an angle θ3 to the inner spine loop 202. The angles θ1, θ2, θ3 preferably each measure between 30° and 90°. More preferably, the angles θ1, θ2, θ3 each measure approximately 60°. As illustrated in Figure 7B, the angle θ1 between the inner spine loop 202 and the outer spine loop 201 measures approximately 35°; the angle θ2 between the outer spine loop 201 and the central spine loop 203 measures approximately 35°; and the angle θ3 between the central spine loop 203 and the inner spine loop 202 measures approximately 120°.

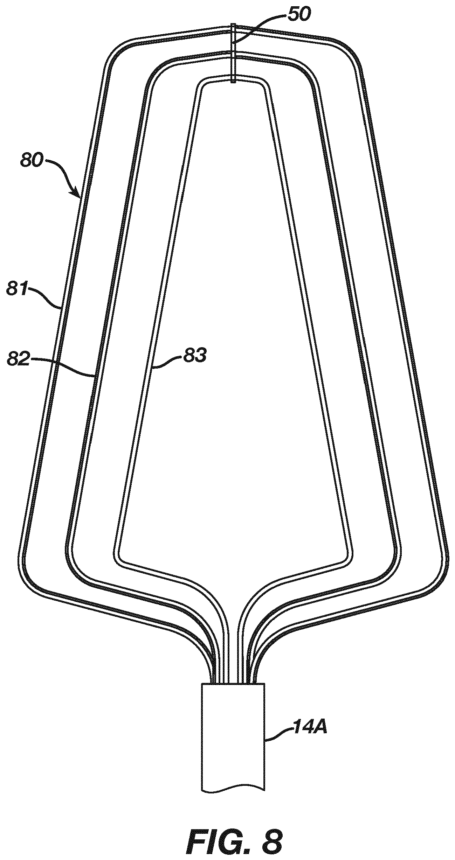

Figure 8 is an illustration of a support frame structure 80 of the end effector 200. The support frame structure 80 includes a first support frame 81, a second support frame 82, and a third support frame 83. Each of the spine loops 201, 202, 203 respectively can include a support frame 81, 82, 83 extending through a respective spine loop. Each support frame 81, 82, 83 can be affixed to the distal portion 14A of the elongated shaft 9 where each end of the respective pair of ends of the respective spine loop 201, 202, 203 is affixed to the distal portion 14A of the elongates shaft 9. Each of the respective support frames 81, 82, 93 can define a respective looped path of its respective spine loop 201, 202, 203 when the end effector 200 is in an unconstrained configuration including the first configuration and the second configuration. Each of the respective support frames 81, 82, 83 can include a respective cross sectional shape orthogonal to the respective looped path, each of the respective cross-sectional shapes varying along the respective looped path. Other example end effectors disclosed herein, including end effectors 100, 300 illustrated in Figures 1-4, 9A, and 9B, can include a similar support frame structure as understood by a person skilled in the pertinent art.

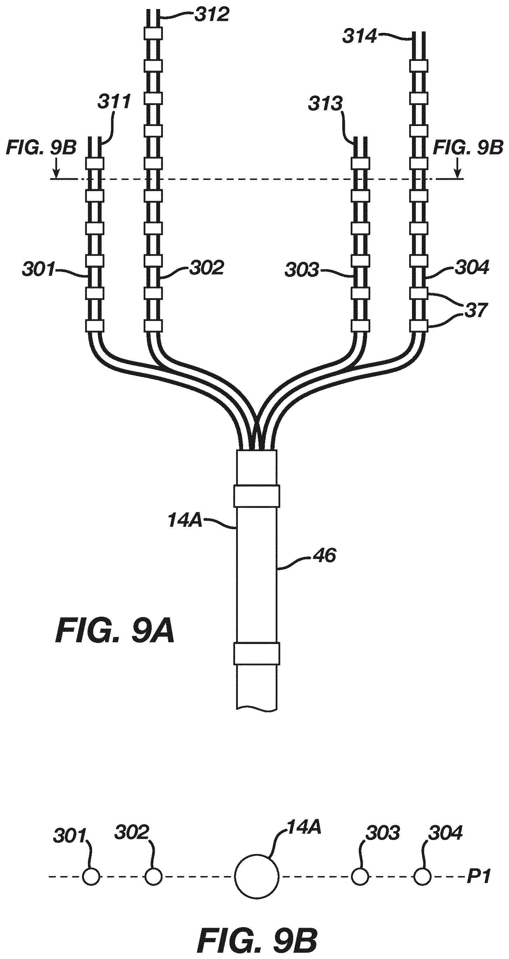

Figure 9A is an illustration of another alternative end effector 300 having spines 301, 302, 303, 304 with free ends 311, 312, 313, 314 in a first configuration. The end effector 300 illustrated in Figure 9A can be used in place of the end effector 100 illustrated in Figure 1 to modify the catheter 10 illustrated in Figure 1.

Figure 9B is an illustration showing a simplified cross-sectional view of the end effector 300 as indicated in Figure 9A. The position of the spines 301, 302, 303, 304 is illustrated with respect to the distal portion 14A of the shaft 9. The spines 301, 302, 303, 304 are aligned with a plane P1.

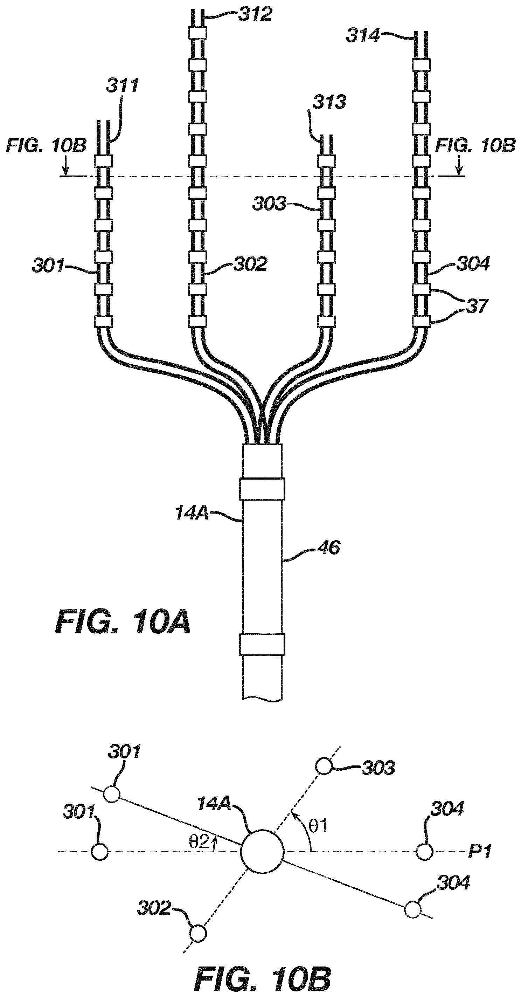

Figure 10A is an illustration of the end effector 300 illustrated in Figure 9A in a second configuration. As illustrated, inner spines 302, 303 are rotated an angle θ1 out of plane P1.

Figure 10B is an illustration showing a simplified cross-sectional view of the end effector 300 as indicated in Figure 10A. As illustrated in Figure 10B, outer spines 301, 304 can optionally be rotated an angle θ2 out of plane P1. In one example, the inner spines 302, 303 can be rotated an angle θ1 that is between approximately 30° and 90° out of plane P1 while the outer spines 301, 304 remain in plane P1. In another example, the outer spines 301, 304 can be rotated an angle θ2 that is between approximately 30° and 90° out of plane P1 while the inner spines 302, 303 remain in plane P1. In another example, all spines 301, 302, 303, 304 can be rotated out of plane P1 so that the spines are at an angle to adjacent spines that is between approximately 30° and 90°. Preferably, spines 301, 302, 303, 304 are at an angle of approximately 90° to adjacent spines.

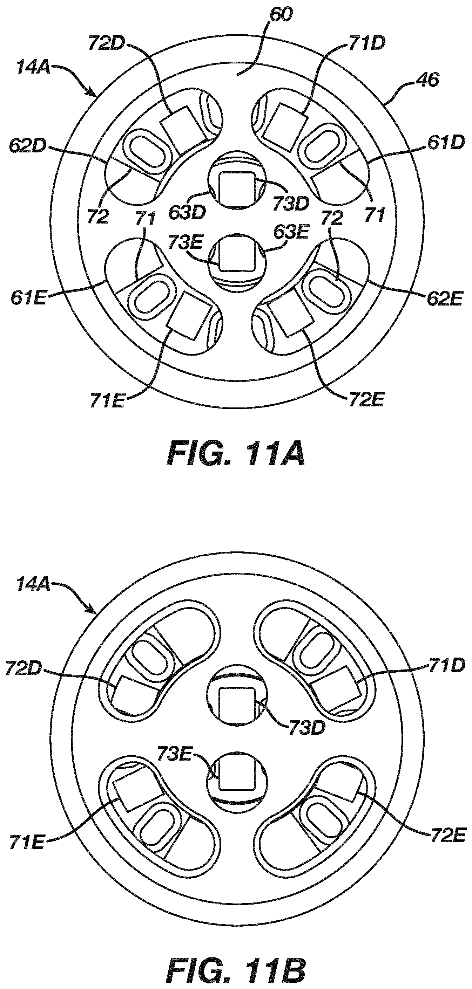

The end effectors 100, 200 illustrated in Figures 1-4 and 9A-B can be modified to include free ends similar to the free ends 311, 312, 313, 314 illustrated in Figures 10A-B and 11A-B. The end effector 300 illustrated in Figures 10A-B and 11A-B can be modified to include spine loops similar to the spine loops 101, 102, 103, 201, 202, 203 illustrated in Figures 1-4 and 9A-B. The end effectors 100, 200, 300 illustrated in Figures 1-4, 9A, 9B, 10A-B, and 11A-B can be modified to include additional or fewer spines and/or spine loops and can include an even number of spines as illustrated or an odd number of spines.

Figure 11A is an illustration of a distal end view of the distal portion 14A of the elongated shaft 9 of an example apparatus in a first configuration. The distal portion 14A of the shaft illustrated in Figure 11A can be adapted to receive an example end effector as disclosed herein, including the end effectors 100, 200, 300 illustrated in Figures 1-4, 9A-B, 10A-B, and 11A-B and variations thereof.

The distal portion 14A of the shaft 9 can include a distal cap 60 with openings 61D, 61E, 62D, 62E, 63D, 63D as illustrated in Figure 6A.

The distal portion can further include a first rotating frame 71 and a second rotating frame 72. The first rotating frame 71 includes openings 71D, 71E for a support frame that extends through a first pair of spines. In one example, a support frame of an outer spine loop is received by the openings 71D, 71E. In another example, support frames through spines having free ends are received by the openings 71D, 71E. The second rotating frame 72 includes openings 72D, 72E for a support frame that extends through a second pair of spines. In one example, a support frame of an inner spine loop is received by the openings 72D, 72E. In another example, support frames through spines having free ends are received by the openings 72D, 72E.

The distal portion 14A can further include a center column 73. The first and second rotating frames 71, 72 can be configured to rotate about the center column 73. The center column 73 can include openings 73D, 73E for a support frame. In one example, a support frame of a central spine loop is received by the openings 73D, 73E. In another example, support frames through spines having free ends are received by the openings 73D, 73E.

The distal portion 14A as illustrated, is configured to receive an end effector having six spines. In one example, one or more of the support frame openings 71D, 71E, 72D, 72E, 73D, 73E are left empty, for instance to accommodate an end effector having fewer than six spines. In another example, the first rotating frame 71, second rotating frame 72, or center column 73 are omitted to accommodate an end effector having four or fewer spines; in such an example, the non-omitted features can be modified as understood by a person skilled in the pertinent art. For instance, a rotating frame and the distal end cap 60 can be modified to allow spines to rotate through a larger angle of rotation about the longitudinal axis.

Figure 11B is an illustration of a distal end view of the distal portion of the elongated shaft of the example apparatus illustrated in Figure 11A in a second configuration. The first rotating frame 71 and the second rotating frame 72 are oppositely rotated about the central column 73.

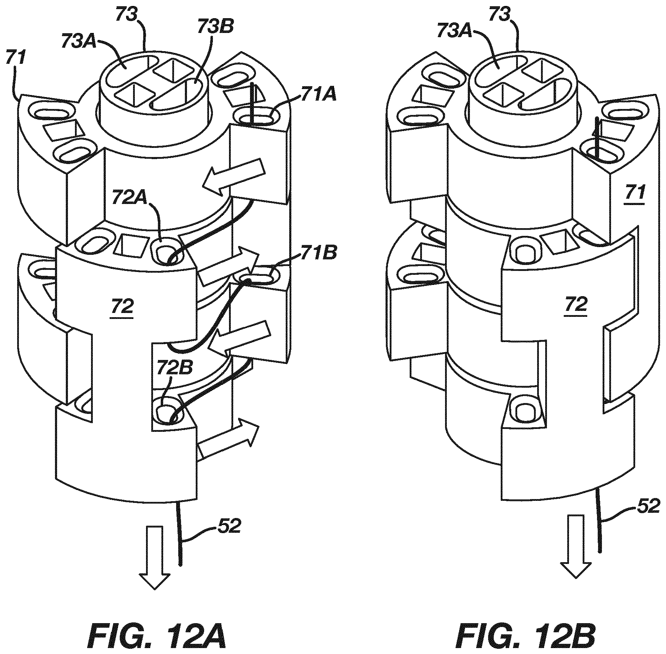

Figure 12A is an illustration of rotating frames 71, 72 of the distal portion 14A of the elongated shaft 9 in the first configuration. Each of the rotating frames 71, 72 can include pull wire openings 71A, 71B, 72A, 71B. The catheter 10 can include one or more pull wires 52 extending from the distal portion 14A of the shaft 9 to the handle 16. The pull wire 52 can be actuated (i.e. pulled and/or released) by the handle 16. The pull wire 52 is elongated in Figure 12A. Arrows in Figure 12A indicate movement of the rotating frames 71, 72 when the pull wire 52 is retracted.

Figure 12B is an illustration of rotating frames 71, 72 of the distal portion 14A of the elongated shaft 9 in the second configuration. The pull wire 52 can be pulled to an approximately linear configuration to align pull wire openings 71A, 71B, 72A, 71B.

The catheter 10 can include additional pull wires which extend through additional pull wire openings illustrated in Figures 12A and 12B positioned similarly to the pull wire openings 71A, 71B, 72A, 71B illustrated with the pull wire 52 extending therethrough. The additional pull wires can function to aid movement of the end effector from the first configuration (Figure 12A) to the second configuration (Figure 12B) and/or to facilitate movement of the end effector from the second configuration (Figure 12B) to the first configuration (Figure 12A). Alternatively, a spring can be implemented with a pull wire to ensure that the catheter can be bias towards either the planar shape or the spherical shape. In yet a further embodiment of the invention is one in which no pull wire is necessary and reliance is instead upon the shape memory effect to ensure that the catheter is heat activated into transform from the planar to the three-dimensional profile (e.g. a cylinder or cone) during use inside a body. In such embodiment, the catheter can be forced into a planar shape by manipulation of the catheter by the operator without reliance on pull wires or spring force.

The center column 73 can include one or more lumens 73A to accommodate electrical conductors which make electrical contact to the electrodes 37 of the end effector.

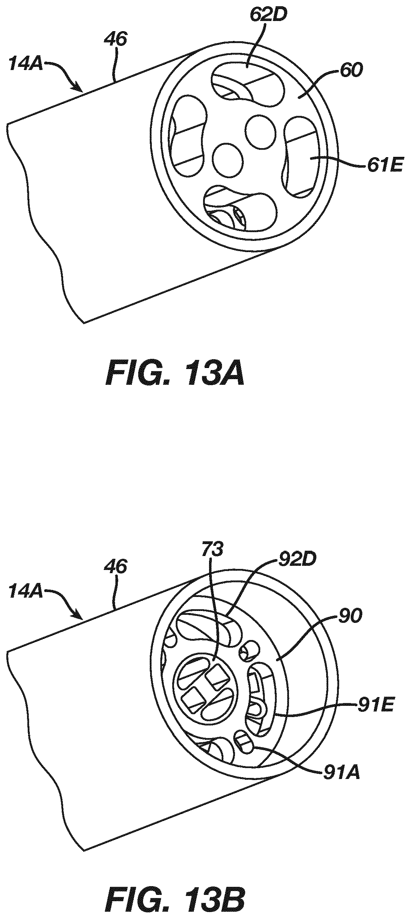

Figure 13A is an illustration of the distal portion 14A of the elongated shaft 9 in a perspective view.

Figure 13B is an illustration of the distal portion 14A of the elongated shaft 9 in Figure 13A with the distal cap 60 removed. The distal cap 60 can be inset into the connector tubing 46. The distal portion 14A can include an anchor cap 90. A distal end of the pull wire 52 can be affixed to the anchor cap 90 at an anchor point 91A. The anchor point 91A can include an opening that the distal end of the pull wire 52 can extend into and can be welded glued, or otherwise affixed at the openings. The anchor cap 90 can include arcuate openings 92D, 91E which align to corresponding arcuate openings 62D, 61E of the distal cap 60. The anchor cap 90 can also include an opening sized to receive a distal portion of the center column 73.

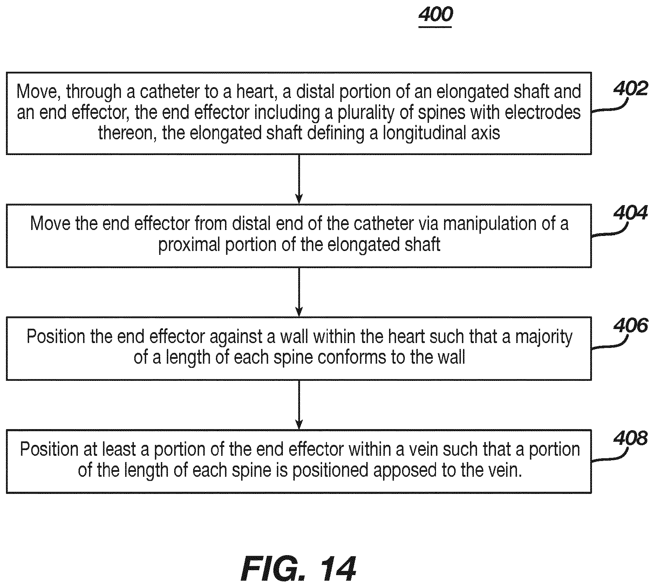

Figure 14 is an illustration of an example flow diagram of a method 400 for mapping electrical signals through cardiac anatomy. At step 402, a distal portion of an elongated shaft and an end effector can be moved through a catheter to a heart. The distal portion can be positioned so that it extends distally from the distal portion of the elongated shaft. The end effector can include a plurality of spines with electrodes thereon, the elongated shaft can define a longitudinal axis. The end effector can be configured similarly to end effectors disclosed herein and variations thereof as understood by a person skilled in the pertinent art. The distal portion of the elongated shaft can be configured similarly to distal portions disclosed herein and variations thereof as understood by a person skilled in the pertinent art.

At step 404, the end effector can be moved from a distal end of the catheter via manipulation of a proximal portion of the elongated shaft.

At step 406, the end effector can be positioned against a wall within the heart such that a majority of a length of each spine conforms to the wall. The end effector can be positioned similar to as illustrated in Figure 3 and variations thereof as understood by a person skilled in the pertinent art.

At step 408, the end effector can be positioned such that at least a portion of the end effector is within a vein and a portion of the length of each spine is positioned apposed to the vein. The end effector can be positioned similar to as illustrated in Figure 4 and variations thereof as understood by a person skilled in the pertinent art. In some examples, the end effector can include an outer pair of spines, an inner pair of spines, and a central pair of spines; in such examples it is preferred that at least the outer pair of spines and the inner pair of spines have portions positioned apposed to the vein. More preferably, portions of the outer pair of spines, the inner pair of spines, and the central pair of spines have portions positioned apposed to the vein.

The method 400 can further include additional steps as otherwise disclosed herein.

### EMBODIMENTS

1. An apparatus comprising:
   an elongated shaft comprising a proximal portion and a distal portion, the elongated shaft configured to be manipulated at the proximal portion to position the distal portion into a heart of a patient, the elongated shaft defining a longitudinal axis of the apparatus;
   a handle affixed to the proximal portion of the shaft; and
   an end effector disposed proximate the distal portion of the elongated shaft, the end effector comprising a plurality of spine pairs each spine includes electrodes disposed thereon, the plurality of spine pairs comprising an outer pair of spines and an inner pair of spines such that in a first configuration, the outer pair of spines are contiguous to a plane with the inner pair of spines, such that in a second configuration, one of the inner pair of spines or the outer pair of spines are rotated about the longitudinal axis so that the one pair of spines is out of alignment with the other pair of spines.
2. The apparatus of embodiment 1, in which each pair of spines includes a connecting member to define a spine loop so that the plurality of spine pairs define a plurality of spine loops,
   spine loops, at least one of the plurality of spine loops being rotatable about the longitudinal axis.
3. The apparatus of embodiment 2,
   each of the spine loops comprising a substantially rectangular shape.
4. The apparatus of embodiment 2,
   each of the spine loops comprising a substantially trapezoidal shape having a distal width wider than a proximal width.
5. The apparatus of embodiment 2,
   the plurality of spine loops comprising three spine loops, and
   at least two of the three spine loops being rotatable about the longitudinal axis by manipulation of the handle.
6. The apparatus of embodiment 2,
   the end effector further comprising a flexible linkage affixed to the plurality of spine loops at a distal end of the end effector along the longitudinal axis.
7. The apparatus of embodiment 2,
   the end effector further comprising a linkage affixed to a distal end of at least one of the spine loops of the plurality of spine loops, extending along the longitudinal axis, and affixed to the distal portion of the elongated shaft.
8. The apparatus of embodiment 1,
   the end effector comprising:
   a first spine loop comprising the outer pair of spines, the first spine loop defining an outer perimeter of the end effector when the plurality of spines are in the first configuration,
   a second spine loop comprising the inner pair of spines, the inner pair of spines being positioned between the outer pair of spines when the plurality of spines are in the first configuration, and
   a third spine loop comprising a central pair of spines of the plurality of spines, the central pair of spines being positioned between the inner pair of spines when the plurality of spines are in the first configuration.
9. The apparatus of embodiment 8,
   the first spine loop being configured to rotate between 30° and 90° about the longitudinal axis, and
   the second spine loop being configured to rotate between -30° and -45° about the longitudinal axis.
10. The apparatus of embodiment 8,
   in the second configuration, the first spine loop being at an angle of approximately 60° to the second spine loop, the second spine loop being at an angle of approximately 60° to the third spine loop, and the third spine loop being at an angle of approximately 60° to the first spine loop.
11. The apparatus of embodiment 1,
   the spines being positioned in the second configuration when the end effector is in free space, and
   the spines being movable to the first configuration when the end effector is pressed against a planar surface.
12. The apparatus of embodiment 1, further comprising:
   a pull wire extending from the handle to the distal portion of the elongated shaft such that manipulation of the handle moves the pull wire and causes the plurality of spines to move between the second configuration and the first configuration.
13. The apparatus of embodiment 12, further comprising:
   a first rotating frame disposed in the distal portion of the elongated shaft and affixed to a first spine of the plurality of spines, the first rotating frame and the first spine being configured to rotate about the longitudinal axis in response to movement of the pull wire.
14. The apparatus of embodiment 13, further comprising:
   a second rotating frame disposed in the distal portion of the elongated shaft and affixed to proximal ends of a second spine loop,
   the first rotating frame being affixed to proximal ends of a first spine loop,
   the first spine loop comprising the first spine and a second spine of the outer pair of spines,
   the first rotating frame and the first spine loop being configured to rotate about the longitudinal axis in response to movement of the pull wire,
   the second spine loop comprising the inner pair of spines,
   the second rotating frame and the second spine loop being configured to rotate about the longitudinal axis in response to movement of the pull wire, and
   the first rotating frame and the first spine loop being configured to rotate oppositely about the longitudinal axis in relation to rotation of the second rotating frame and the second spine loop about the longitudinal axis.
15. The apparatus of embodiment 14,
   the first spine loop comprising a first support frame affixed to the first rotating frame, and
   the second spine loop comprising a second support frame affixed second rotating frame.
16. The apparatus embodiment 14, further comprising:
   a distal cap affixed at the distal portion of the elongated shaft, distal of the first rotating frame and the second rotating frame,
   wherein a distal end of the pull wire is affixed to the distal cap,
   wherein the pull wire is threaded through a first pull wire lumen of the first rotating frame,
   wherein the pull wire is threaded through a second pull wire lumen of the second rotating frame, and
   wherein tension in the pull wire moves the first pull wire lumen and the second pull wire lumen into alignment.
17. The apparatus of embodiment 16, further comprising:
   a plurality of pull wires each affixed to the distal cap and each threaded through a first respective pull wire lumen of the first rotating frame and a second respective pull wire lumen of the second rotating frame,
   wherein the plurality of pull wires are configured to move the plurality of spines from the first configuration to the second configuration and from the second configuration to the first configuration.
18. The apparatus of embodiment 14, further comprising:
   a center column disposed in the distal portion of the elongated shaft and affixed to proximal ends of a third spine loop, the third spine loop comprising a central pair of spines, the first rotating frame and/or the second rotating frame being movable to rotate about the center column in response to movement of the pull wire.
19. The apparatus of embodiment 18, further comprising:
   a plurality of electrical conductors extending through the elongated shaft, through the center column, and electrically connected to the electrodes.
20. The apparatus of embodiment 1, the distal portion of the elongated shaft being configured to deflect in relation to longitudinal axis in response to manipulation of the handle.

## Claims

1. An apparatus comprising:
an elongated shaft comprising a proximal portion and a distal portion, the elongated shaft configured to be manipulated at the proximal portion to position the distal portion into a heart of a patient, the elongated shaft defining a longitudinal axis of the apparatus;
a handle affixed to the proximal portion of the shaft; and
an end effector disposed proximate the distal portion of the elongated shaft, the end effector comprising a plurality of spine pairs each spine includes electrodes disposed thereon, the plurality of spine pairs comprising an outer pair of spines and an inner pair of spines such that in a first configuration, the outer pair of spines are contiguous to a plane with the inner pair of spines, such that in a second configuration, one of the inner pair of spines or the outer pair of spines are rotated about the longitudinal axis so that the one pair of spines is out of alignment with the other pair of spines.

2. The apparatus of claim 1, in which each pair of spines includes a connecting member to define a spine loop so that the plurality of spine pairs define a plurality of spine loops,
spine loops, at least one of the plurality of spine loops being rotatable about the longitudinal axis.

3. The apparatus of claim 2,
each of the spine loops comprising a substantially rectangular shape, or
each of the spine loops comprising a substantially trapezoidal shape having a distal width wider than a proximal width.

4. The apparatus of claim 2,
the plurality of spine loops comprising three spine loops, and
at least two of the three spine loops being rotatable about the longitudinal axis by manipulation of the handle.

5. The apparatus of claim 2,
the end effector further comprising a flexible linkage affixed to the plurality of spine loops at a distal end of the end effector along the longitudinal axis.

6. The apparatus of claim 2,
the end effector further comprising a linkage affixed to a distal end of at least one of the spine loops of the plurality of spine loops, extending along the longitudinal axis, and affixed to the distal portion of the elongated shaft.

7. The apparatus of claim 1,
the end effector comprising:
a first spine loop comprising the outer pair of spines, the first spine loop defining an outer perimeter of the end effector when the plurality of spines are in the first configuration,
a second spine loop comprising the inner pair of spines, the inner pair of spines being positioned between the outer pair of spines when the plurality of spines are in the first configuration, and
a third spine loop comprising a central pair of spines of the plurality of spines, the central pair of spines being positioned between the inner pair of spines when the plurality of spines are in the first configuration.

8. The apparatus of claim 7,
the first spine loop being configured to rotate between 30° and 90° about the longitudinal axis, and
the second spine loop being configured to rotate between -30° and -45° about the longitudinal axis.

9. The apparatus of claim 7,
in the second configuration, the first spine loop being at an angle of approximately 60° to the second spine loop, the second spine loop being at an angle of approximately 60° to the third spine loop, and the third spine loop being at an angle of approximately 60° to the first spine loop.

10. The apparatus of claim 1,
the spines being positioned in the second configuration when the end effector is in free space, and
the spines being movable to the first configuration when the end effector is pressed against a planar surface.

11. The apparatus of claim 1, further comprising:
a pull wire extending from the handle to the distal portion of the elongated shaft such that manipulation of the handle moves the pull wire and causes the plurality of spines to move between the second configuration and the first configuration, optionally:
further comprising:
a first rotating frame disposed in the distal portion of the elongated shaft and affixed to a first spine of the plurality of spines, the first rotating frame and the first spine being configured to rotate about the longitudinal axis in response to movement of the pull wire.

12. The apparatus of claim 11, further comprising:
the first rotating frame disposed in the distal portion of the elongated shaft and affixed to the first spine of the plurality of spines, the first rotating frame and the first spine being configured to rotate about the longitudinal axis in response to movement of the pull wire;
a second rotating frame disposed in the distal portion of the elongated shaft and affixed to proximal ends of a second spine loop,
the first rotating frame being affixed to proximal ends of a first spine loop,
the first spine loop comprising the first spine and a second spine of the outer pair of spines,
the first rotating frame and the first spine loop being configured to rotate about the longitudinal axis in response to movement of the pull wire,
the second spine loop comprising the inner pair of spines,
the second rotating frame and the second spine loop being configured to rotate about the longitudinal axis in response to movement of the pull wire, and
the first rotating frame and the first spine loop being configured to rotate oppositely about the longitudinal axis in relation to rotation of the second rotating frame and the second spine loop about the longitudinal axis.

13. The apparatus of claim 12,
the first spine loop comprising a first support frame affixed to the first rotating frame, and
the second spine loop comprising a second support frame affixed second rotating frame.

14. The apparatus claim 12, further comprising:
a distal cap affixed at the distal portion of the elongated shaft, distal of the first rotating frame and the second rotating frame,
wherein a distal end of the pull wire is affixed to the distal cap,
wherein the pull wire is threaded through a first pull wire lumen of the first rotating frame,
wherein the pull wire is threaded through a second pull wire lumen of the second rotating frame, and
wherein tension in the pull wire moves the first pull wire lumen and the second pull wire lumen into alignment, optionally:
further comprising:
a plurality of pull wires each affixed to the distal cap and each threaded through a first respective pull wire lumen of the first rotating frame and a second respective pull wire lumen of the second rotating frame,
wherein the plurality of pull wires are configured to move the plurality of spines from the first configuration to the second configuration and from the second configuration to the first configuration.

15. The apparatus of claim 12, further comprising:
a center column disposed in the distal portion of the elongated shaft and affixed to proximal ends of a third spine loop, the third spine loop comprising a central pair of spines, the first rotating frame and/or the second rotating frame being movable to rotate about the center column in response to movement of the pull wire, optionally:
further comprising:
a plurality of electrical conductors extending through the elongated shaft, through the center column, and electrically connected to the electrodes.

16. The apparatus of claim 1, the distal portion of the elongated shaft being configured to deflect in relation to longitudinal axis in response to manipulation of the handle.
